(19) Europäisches Patentamt
European Patent Office
Office européen des brevets

(11) Veröffentlichungsnummer: **0 099 339**
**B1**

(12) # EUROPÄISCHE PATENTSCHRIFT

(45) Veröffentlichungstag der Patentschrift: **07.01.87**

(21) Anmeldenummer: **83810314.1**

(22) Anmeldetag: **11.07.83**

(51) Int. Cl.⁴: **C 07 D 405/12**, C 07 D 409/12, C 07 D 411/12, C 07 D 401/12, A 01 N 47/36 // C07D215/36, C07D307/79, C07D307/83, C07D307/88, C07D311/58, C07D311/70, C07D317/62, C07D317/66, C07D319/18, C07D327/06, C07D333/54, C07D335/06

(54) **Anellierte N-Phenylsulfonyl-N'-pyrimidinyl- und -triazinylharnstoffe.**

(30) Priorität: **16.07.82 CH 4357/82**

(43) Veröffentlichungstag der Anmeldung: **25.01.84 Patentblatt 84/4**

(45) Bekanntmachung des Hinweises auf die Patenterteilung: **07.01.87 Patentblatt 87/2**

(84) Benannte Vertragsstaaten: **AT BE CH DE FR GB IT LI NL SE**

(56) Entgegenhaltungen:
**EP - A - 0 045 196**
**EP - A - 0 051 465**
**EP - A - 0 079 683**
**EP - A - 0 082 681**
**US - A - 4 127 405**

**CHIMIE THERAPEUTIQUE, Nr. 6, November/Dezember 1973, Seiten 659-668, Paris, FR; H. BREUER u.a.: "Potential antidiabetic agents. 1. Indan-, indole-, indoline-, coumaran- and dihydrothianaphthene sulfonylureas und sulfonylsemicarbazides"**

(73) Patentinhaber: **CIBA-GEIGY AG, Klybeckstrasse 141, CH-4002 Basel (CH)**

(72) Erfinder: **Ehrenfreund, Josef, Dr., Amselstrasse 11, CH-4123 Allschwil (CH)**
Erfinder: **Föry, Werner, Dr., Benkenstrasse 65, CH-4054 Basel (CH)**
Erfinder: **Meyer, Willy, Talweg 49, CH-4125 Riehen (CH)**
Erfinder: **Töpfl, Werner, Dr., Dorneckstrasse 68, CH-4143 Dornach (CH)**

**EP 0 099 339 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue, herbizid wirksame und pflanzenwuchsregulierende anellierte N-Phenylsulfonyl-N'-pyrimidinyl- und -triazinyl-harnstoffe, Verfahren zu ihrer Herstellung, sie als Wirkstoffe enthaltende Mittel sowie deren Verwendung zur Bekämpfung von Unkräutern, vor allem selektiv in Nutzpflanzenkulturen oder zum Regulieren und Hemmen des Pflanzenwachstums. Darüber hinaus betrifft die Erfindung auch als Zwischenprodukte hergestellte neue anellierte Phenylsulfonylisocyanate, -thioisocyanate, Phenylsulfonylcarbamate, Phenylsulfonamide sowie Phenylsulfonsäurechloride.

Die erfindungsgemässen anellierten N-Phenylsulfonyl-N'-pyrimidinyl- und -triazinyl-harnstoffe entsprechen der allgemeinen Formel I

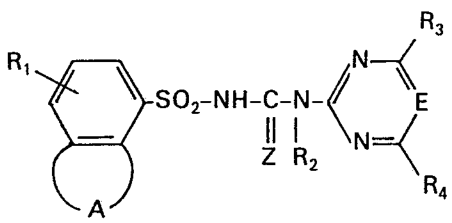

(I),

worin

Z Sauerstoff oder Schwefel,

E Stickstoff oder =CH–,

$R_1$ Wasserstoff, Halogen, Nitro, $C_1$–$C_4$-Alkyl, $C_1$–$C_4$-Halogenalkyl, $C_1$–$C_4$-Alkoxy, $C_1$–$C_4$-Halogenalkoxy, $C_1$–$C_4$-Alkoxycarbonyl, $C_1$–$C_4$-Alkylthio, $C_1$–$C_4$-Alkylsulfinyl, $C_1$–$C_4$-Alkylsulfonyl oder $C_2$–$C_5$-Alkoxyalkoxy,

$R_2$ Wasserstoff, $C_1$–$C_4$-Alkyl oder $C_1$–$C_3$-Alkoxy,

$R_3$ und $R_4$ unabhängig voneinander Wasserstoff, $C_1$–$C_4$-Alkyl, $C_1$–$C_4$-Alkoxy, $C_1$–$C_4$-Halogenalkoxy, $C_1$–$C_4$-Halogenalkylthio, $C_1$–$C_4$-Alkylthio, Halogen, $C_2$–$C_5$-Alkoxyalkyl, $C_2$–$C_5$-Alkoxyalkoxy oder $-NR_5R_6$, wobei $R_5$ und $R_6$ für Wasserstoff oder $C_1$–$C_4$-Alkyl stehen, bedeuten und die Brücke A zusammen mit dem sie tragenden Phenylkern ein 3,4-Dihydro-2,2-dioxo-1,2-benzoxathiin-System bildet, das an der Brücke A gegebenenfalls durch $C_1$–$C_4$-Alkyl substituiert ist, sowie den Salzen dieser Verbindungen.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise im niederländischen Patent Nr. 121 788, im US-Patent Nr. 4 127 405 und in den europäischen Patentanmeldungen 44 807, 44 808, 45 196, 51 465, 79 683 und 82 681 beschrieben.

In den Definitionen ist unter Alkyl geradkettiges oder verzweigtes Alkyl zu verstehen; z.B. Methyl, Äthyl, n-Propyl, i-Propyl oder die vier isomeren Butyl.

Unter Alkoxy ist zu verstehen: Methoxy, Äthoxy, n-Propyloxy, i-Propyloxy, die vier isomeren Butyloxyreste, n-Amyloxy, i-Amyloxy, 2-Amyloxy oder 3-Amyloxy, insbesondere aber Methoxy, Äthoxy oder i-Propyloxy.

Beispiele für Alkylthio sind Methylthio, Äthylthio, n-Propylthio, i-Propylthio, n-Butylthio oder n-Pentylthio, insbesondere aber Methylthio und Äthylthio.

Beispiele für Alkylsulfinyl sind Methylsulfinyl, Äthylsulfinyl, n-Propylsulfinyl und n-Butylsulfinyl, insbesondere aber Methylsulfinyl und Äthylsulfinyl.

Beispiele für Alkylsulfonyl sind Methylsulfonyl, Äthylsulfonyl oder n-Propylsulfonyl, insbesondere aber Methylsulfonyl und Äthylsulfonyl.

Unter Halogen in den Definitionen sowie als Teil in Halogenalkoxy sind Fluor, Chlor und Brom, vorzugsweise jedoch Fluor und Chlor zu verstehen.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Äthylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Unter den Verbindungen der Formel I sind diejenigen bevorzugt, in denen entweder

a) Z Sauerstoff ist oder

b) $R_1$ Wassertoff, Halogen oder C1-$C_4$-Alkoxycarbonyl ist oder

c) $R_2$ für Wasserstoff steht oder

d) $R_3$ und $R_4$ unabhängig voneinander $C_1$–$C_4$-Alkyl, $C_1$–$C_4$-Alkoxy, Di-$C_1$–$C_4$-alkylamino,$C_1$–$C_4$-Halogenalkoxy oder Halogen bedeuten und zusammen höchstens 4 Kohlenstoffatome enthalten.

Eine besonders bevorzugte Gruppe von Wirkstoffen der Formel I bilden diejenigen Verbindungen, in denen Z Sauerstoff, $R_1$ Wasserstoff, Halogen oder $C_1$–$C_4$-Alkoxycarbonyl, $R_2$ Wasserstoff, $R_3$ und $R_4$ unabhängig voneinander $C_1$–$C_4$-Alkyl, $C_1$–$C_4$-Alkoxy, Di-$C_1$–$C_4$-alkylamino, $C_1$–$C_4$-Halogenalkoxy oder Halogen, welche zusammen

höchstens 4 Kohlenstoffatome enthaltn, bedeuten.

Die Herstellung der Verbindungen der Formel I erfolgt in einem inerten, organischen Lösungsmittel.

Nach einem ersten Verfahren werden die Verbindungen der Formel I erhalten, indem man ein anelliertes Phenylsulfonamid der Formel II

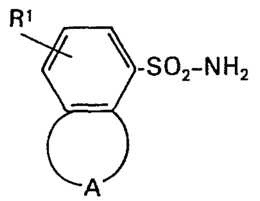

(II),

worin $R^1$ und A die unter Formel I gegebene Bedeutung haben, in Gegenwart einer Base mit einem N-Pyrimidinyl- oder -triazinylcarbamat der Formel III

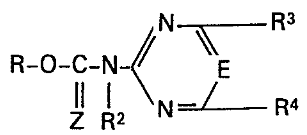

(III),

worin E, $R^2$, $R^3$, $R_4$ und Z die unter Formel I gegebene Bedeutung haben und R für Phenyl, Alkyl oder substituiertes Phenyl steht, umsetzt.

Nach einem zweiten Verfahren gelangt man zu Verbindungen der Formel I, indem man ein anelliertes Phenylsulfonylisocyanat oder -isothiocyanat der Formel IV

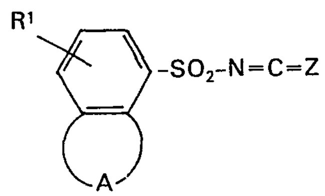

(IV),

worin A, $R^1$ und Z die unter Formel I gegebene Bedeutung haben, gegebenenfalls in Gegenwart einer Base mit einem Aminopyrimidin oder -triazin der Formel V

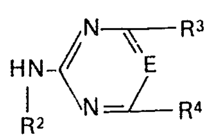

(V),

worin E, $R^2$, $R^3$ und $R^4$ die unter Formel I gegebene Bedeutung haben, umsetzt.

Schliesslich kann man die Verbindungen der Formel I auch erhalten, indem man ein anelliertes N-Phenylsulfonylcarbamat der Formel VI

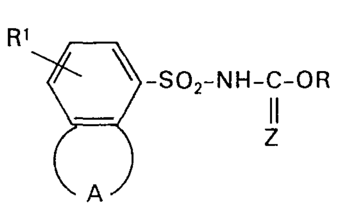

(VI),

worin A, $R^1$ und Z die unter Formel I gegebene Bedeutung haben und R für Phenyl, Alkyl oder substituiertes Phenyl steht, mit einem Aminopyrimidin oder -triazin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen des Lösungsmittels.

Die Umsetzungen zu Verbindungen der Formel I werden mit Vorteil in aprotischen, inerten organischen Lösungsmitteln vorgenommen.

Solche Lösungsmittel sind Kohlenwasserstoffe wie Benzol, Toluol, Xylol oder Cyclohexan, chlorierte Kohlenwasserstoffe wie Methylenchlorid, Chloroform, Tetrachlorkohlenstoff, oder Chlorbenzol, Äther wie Diäthyläther, Äthylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen -20° und +120°C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt weden. Als Basen sind insbesondere tertiäre Amine wie Trimethylamin, Triäthylamin, Chinuclidin, 1,4-Diazabicyclo-(2,2,2)-octan, 1,5-Diazabicyclo(4,3,0)non-5-en oder 1,5-Diazabicyclo(5,4,0)un-dec-7-en geeignet. Als Basen können aber auch anorganische Basen wie Hydride wie Natrium- oder Calciumhydrid, Hydroxide wie Natrium- oder Kaliumhydroxid, Carbonate wie Natrium- und Kaliumcarbonat oder Hydrogencarbonate wie Kalium- und Natriumhydrogencarbonat, verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Äther, aromatischen Kohlenwasserstoffen oder chlorierten Kohlenwasserstoffen gereinigt werden.

Die Zwischenprodukte der Formeln II, IV und VI sind neu und wurden speziell für die Synthese der Verbindungen der Formel I entwickelt. Sie bilden daher einen Bestandteil der vorliegenden Erfindung.

Die neuen Zwischenprodukte der Formel II können auf verschiedenen Wegen hergestellt werden. So erhält man die Verbindungen der Formel II, indem man Aniline der Formel VII

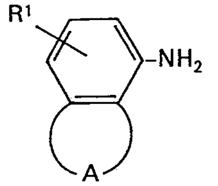

(VII),

worin $R^1$ und A die unter Formel I gegebene Bedeutung haben, diazotiert und die Diazogruppe mit Schwefeldioxid in Gegenwart eines Katalysators wie Kupferchlorid in Salzsäure oder Essigsäure austauscht und das entstandene Phenylsulfonylchlorid der Formel X

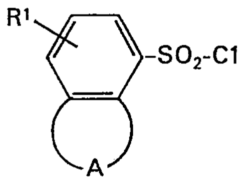

(X),

worin A und $R^1$ die unter Formel I angegebene Bedeutung haben, mit Ammoniumhydroxid-Lösung umsetzt. Die als Ausgangsprodukte verwendeten entsprechenden Anilin-Derivate sind bekannt oder nach bekannten Methoden herstellbar.

Ebenso kann man die Verbindungen der Formel II erhalten, indem man eine Phenylsulfonsäure der Formel VIII

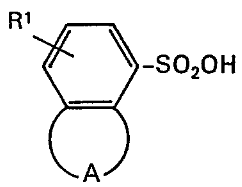

(VIII),

worin $R^1$ und A die unter Formel I gegebene Bedeutung haben, durch Behandeln mit einem Chlorierungsmittel wie $PCl_5$, $POCl_3$, $COCl_2$ oder $SOCl_2$ in das entsprechende Phenylsulfonylchlorid der Formel X überführt und dieses mit Ammoniumhydroxid-Lösung umsetzt.

Weiter kann man die Verbindungen der Formel II erhalten, indem man einen Benzylthioäther der Formel IX

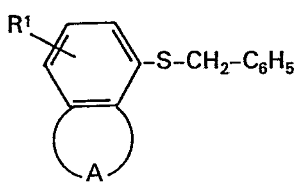

(IX),

worin $R^1$ und A die unter Formel I gegebene Bedeutung haben, mit Chlor behandelt und das entstandene Phenylsulfonylchlorid der Formel X mit Ammoniumhydroxid-Lösung umsetzt. In bestimmten Fällen, z.B. beim Vorliegen einer aktivierten Substitutionsposition, ist eine direkte Sulfochlorierung des Phenylkerns möglich, die durch Umsetzung mit einem Überschuss an Chlorschwefelsäure zu dem entsprechenden anellierten Phenylsulfonylchlorid führt. Entsprechend aktivierte anellierte Benzolderivate sind bekannt.

Die ebenfalls neuen Phenylsulfonylisocyanate der Formel IV können beispielsweise durch Umsetzungen der Sulfonamide der Formel II mit Phosgen in Anwesenheit von Butylisocyanat in einem chlorierten Kohlenwasserstoff als Lösungsmittel, bei Rückflusstemperatur erhalten werden. Ähnliche Darstellungen sind in «Neuere Methoden der präparativen organischen Chemie», Band VI, 211–229, Verlag Chemie, Weinheim, 1970, beschrieben.

Die Isothiocyanate der Formel IV werden durch Behandlung der Sulfonamide der Formel II mit Schwefelkohlenstoff und Kaliumhydroxid und anschliessende Umsetzung des Dikaliumsalzes mit Phosgen erhalten. Solche Verfahren sind in Arch. Pharm. 299, 174 (1966) beschrieben.

Die N-Phenylsulfonylcarbamate der Formel VI werden durch Umsetzung der Sulfonamide der Formel II mit Diphenylcarbonat in Gegenwart einer Base erhalten. Ähnliche Verfahren sind in der japanischen Patentschrift 61 169 erwähnt.

Die ebenfalls neuen anellierten Phenylsulfonylchloride der Formel X wurden ebenfalls speziell für die Synthese der Wirkstoffe der Formel I entwickelt. Sie sind daher ebenfalls Gegenstand der vorliegenden Erfindung.

Die als Ausgangsmaterialien verwendeten Aminopyrimidine und -triazine der Formel V sowie entsprechende Phenylcarbamate der Formel III sind entweder seit langem bekannt oder in der europäischen Patentanmeldung Nr. 70 804 beschrieben oder sie lassen sich nach bekannten Methoden aus dort beschriebenen Verbindungen erhalten.

Die Wirkstoffe der Formel I sind stabile Verbindungen. Ihre Handhabung bedarf keiner vorsorglicher Massnahmen.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei – im Vergleich zu anderen Herbiziden und Wuchsregulatoren – sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als «cover crops» (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die «cover crops» jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Öl- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8–22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4–14)-Äthylenoxid-Adduktes oder Phospholipide in Frage.

Als nicht ionische Tenside kommen in erster Linie Polyglykolätherderivate von aliphatischen

oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate oder Äthylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

«Mc Cutcheon's Detergents and Emulsifiers Annual» MC

Publishing Corp., Ridgewood, New Jersey, 1979.

Sisley and Wood, «Encyclopedia of Surface Active Agents».

Chemical Publishing Co., Inc. New York, 1964.

Die pestiziden Zubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25%, eines Tensides.

Insbesondere setzen sich bevorzugte Formulierungen folgendermassen zusammen: (% = Gewichtsprozent)

Emulgierbare Konzentrate
Aktiver Wirkstoff:
1 bis 20%, bevorzugt 5 bis 10%
oberflächenaktives Mittel:
5 bis 30%, vorzugsweise 10 bis 20%
flüssiges Trägermittel:
50 bis 94%, vorzugsweise 70 bis 85%.

Stäube
Aktiver Wirkstoff:
0,1 bis 10%, vorzugsweise 0,1 bis 1%
festes Trägermittel:
99,9 bis 90%, vorzugsweise 99,9 bis 99%.

Suspensions-Konzentrate
Aktiver Wirkstoff:
5 bis 75%, vorzugsweise 10 bis 50%
Wasser:
94 bis 25%, vorzugsweise 90 bis 30%
oberflächenaktives Mittel:
1 bis 40%, vorzugsweise 2 bis 30%.

Benetzbare Pulver
Aktiver Wirkstoff:
0,5 bis 90%, vorzugsweise 1 bis 80%
oberflächenaktives Mittel:
0,5 bis 20%, vorzugsweise 1 bis 15%
festes Trägermittel:
5 bis 95%, vorzugsweise 15 bis 90%.

Granulate
Aktiver Wirkstoff:
0,5 bis 30%, vorzugsweise 3 bis 15%
festes Trägermittel:
99,5 bis 70%, vorzugsweise 97 bis 85%.

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

Beispiel 1

a) 5-Brom-2,3-dihydro-2-methyl-benzo[b]furan

Zu einem Gemisch von 237 g (1,77 Mol) 2,3-Dihydro-2-methyl-benzo[b]furan, 148,7 g (1,77 Mol) Natriumhydrogencarbonat, 500 ml Methylenchlorid und 500 ml Wasser lässt man unter kräftigem Rühren bei 0° bis 5°C 283 g (1,77 Mol) Brom zutropfen. Nach Beendigung des Bromzusatzes wird das Gemisch für weitere 0,5 Stunden bei 20° bis 25°C gerührt. Die organische Phase wird abgetrennt, über Natriumsulfat getrocknet, eingedampft und im Vakuum fraktioniert. Man erhält 242 g (66% d. Th.) 5-Brom-2,3-dihydro-2-methyl-benzo[b]furan und 77 g (15% d. Th.) 5,6-Dibrom-2,3-dihydro-2-methyl-benzo[b]furan.

b) 5-Brom-2,3-dihydro-2-methyl-7-benzo[b]furanylsulfonsäurechlorid

Zu einer Lösung von 106 g (0,5 Mol) 5-Brom-2,3-dihydro-2-methyl-benzo[b]furan in 300 ml Chloroform lässt man bei −7°C innerhalb von 20 Minuten 470 g (4,03 Mol) Chlorschwefelsäure zutropfen und rührt das Gemisch anschliessend für weitere 15 Minuten bei 20° bis 25°C. Das Reaktionsgemisch wird in Eiswasser aufgenommen, die organische Phase abgetrennt und die wässrige Phase zweimal mit je 500 ml Chloroform extrahiert. Die vereinigten organischen Phasen werden zweimal mit je 250 ml Wasser gewaschen, über Natriumsulfat getrocknet und eingedampft. Als Rückstand erhält man 101 g (65% d. Th.) 5-Brom-2,3-dihydro-2-methyl-7-benzo[b]furanylsulfonsäurechlorid.

c) 5-Brom-2,3-dihydro-2-methyl-7-sulfamoylbenzo[b]furan

Das unter b) erhaltene Rohprodukt wird in 200 ml Äthylacetat gelöst und bei 20° bis 25°C tropfenweise zu 250 ml 25%igem wässrigen Ammoniak zugesetzt. Nachdem die Reaktionsmischung für eine weitere Stunde bei der gleichen Temperatur gerührt worden ist, wird die organische Phase abgetrennt, mit Aktivkohle behandelt, über Natriumsulfat getrocknet und eingedampft. Durch Kristallisation des Rückstands erhält man 75 g (80% d. Th.) 5-Brom-2,3-dihydro-2-methyl-7-sulfamoyl-Benzo[b]furan, Smp. 167–169°C.

d) 2,3-Dihydro-2-methyl-7-sulfamoyl-benzo[b]furan

72,5 g (0,25 Mol) 5-Brom-2,3-dihydro-2-methyl-7-sulfamoyl-benzo[b]furan werden in Gegenwart von 22,4 g (0,25 Mol) Natriumacetat und 7,5 g 5%igem Palladium/Kohle-Katalysator in 750 ml Methanol hydriert. Nach Abtrennen des Katalysators, Abdampfen des Lösungsmittels und Kristallisieren des Rückstandes aus Wasser/Äthanol-Gemisch erhält man 42,3 g (79% d. Th.) 2,3-Dihydro-2-methyl-7-sulfamoyl-benzo[b]furan. Smp. 174–177°C.

e) N-(2,3-Dihydro-2-methyl-benzo[b]furan-7-ylsulfonyl)-N'-(4,6-dimethyl-pyrimidin-2-yl)-harnstoff

Eine Lösung von 5,2 g (0,025 Mol) 2,3-Dihydro-2-methyl-7-sulfamoyl-benzo[b]furan und 6,0 g (0,025 Mol) N-(4,6-Dimethyl-pyrimidin-2-yl)-phenylcarbamat in 50 ml Acetonitril wird mit 3,9 g (0,025 Mol) 1,5-Diazabicyclo(5,4,0)undec-5-en versetzt und für 2 Stunden bei 20° bis 25°C gerührt. Nach Beendigung der Reaktion wird das Gemisch mit Wasser verdünnt, wobei das Produkt auskristallisiert. Der Niederschlag wird abgetrennt, mit Wasser und Hexan gewaschen und im Vakuum über Kaliumhydroxid getrocknet. Man erhält 7,0 g (78% d. Th.) N-(2,3-Dihydro-2-methyl-benzo[b]furan-7-ylsulfonyl)-N'-(4,6-dimethyl-pyrimidin-2-yl)-harnstoff, Smp. 190–193°C.

Beispiel 2

1,3-Benzodioxol-4-sulfonsäureamid

a) 1,3-Benzodioxol-4-sulfonsäurechlorid.

7,5 g 4-Amino-1,3-benzodioxol werden in 23 ml 36%iger Salzsäure suspendiert und unter Kühlung mit Eis mit einer Lösung von 4,06 g Natriumnitrit in 8 ml Wasser diazotiert. Die erhaltene Diazoniumsalzlösung wird tropfenweise einem Gemisch aus 50 ml Eisessig, 5 ml Wasser, 15 g Schwefeldioxid und 2,3 g Kupfer-II-chlorid-hydrat zugesetzt. Die Temperatur wird bis zum vollständigen Zusatz der Diazoniumsalzlösung auf 40°C konstant gehalten. Anschliessend wird das Reaktionsgemisch abgekühlt und mit 500 ml Eiswasser verdünnt. Das anfallende rohe 1,3-Benzodioxol-4-sulfonsäurechlorid wird abgetrennt und getrocknet, Smp. 121–124°C.

b) 1,3-Benzodioxol-4-sulfonsäureamid.

Das unter a) erhaltene 1,3-Benzodioxol-4-sulfonsäurechlorid wird in 20 ml Äthylacetat gelöst und bei 20–25°C tropfenweise zu 25 ml 25%igem wässrigem Ammoniak zugesetzt. Diese Mischung wird für eine Stunde bei der gleichen Temperatur gerührt. Die organische Phase wird abgetrennt, getrocknet und eingedampft. Man erhält so 1,3-Benzodioxol-4-sulfonsäureamid.

Beispiel 3

2,3-Dihydro-2,2-dimethyl-benzo[b]furan-7-yl-sulfonylisocyanat

a) N-(2,3-Dihydro-2,2-dimethyl-benzo[b]furan-7-yl-sulfonyl)-N'-methylharnstoff.

Zu 9,9 g (0,0436 Mol) 2,3-Dihydro-2,2-dimethyl-7-sulfamoyl-benzo[b]furan in Dioxan (Verb. 1.4.) werden 6,54 ml 1,5-Diazabicyclo(5,4,0)-undec-5-en zugefügt. Anschliessend lässt man unter Kühlung mit Eis 2,65 ml Methylisocyanat zutropfen. Nach Rühren für 2 Stunden bei 20–25°C wird das Gemisch mit Wasser verdünnt, mit 10 ml 5%iger Sodalösung versetzt und filtriert. Beim Ansäuern des Filtrates fallen 1,1 g (89,5%) N-(2,3-Dihydro-2,2-dimethyl-benzo[b]furan-7-yl-sulfonyl)-N'-methylharnstoff an.

b) 2,3-Dihydro-2,2-dimethyl-benzo[b]furan-7-yl-sulfonylisocyanat.

8 g N-(2,3-Dihydro-2,2-dimethyl-benzo[b]furan-7-yl-sulfonyl)-N'-methylharnstoff werden in 400 ml absolutem Chlorbenzol dispergiert und bei etwa 130°C mit Phosgen gesättigt. Dabei erhält man eine klare Lösung. Anschliessend wird das Lösungsmittel im Vakuum unter Feuchtigkeitsschluss abdestilliert. Das als Öl anfallende 2,3-Dihydro-2,2-dimethyl-benzo[b]furan-7-yl-sulfonylisocyanat kann ohne weitere Reinigung zur Darstellung der erfindungsgemässen anellierten N-Phenylsulfonyl-N'-pyrimidinyl und -triazinylharnstoffe der Formel I weiterverwendet werden.

In analoger Weise erhält man die in den folgenden Tabellen aufgelisteten Zwischen- und Endprodukte.

Die in der Tabelle 1 zusätzlich aufgeführten Ringsystem-Nummern beziehen sich nur auf den anellierten Phenylring und werden für den gleichen Substituenten in den nachfolgenden Tabellen verwendet.

Tabelle 1

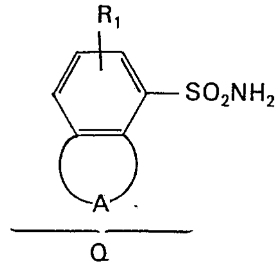

| Verb. Nr. | Ringsystem Nr. | Q | Smp. [°C] |
|---|---|---|---|
| 1.1 | $Q_1$ | 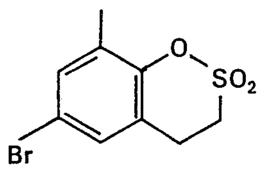 | 265-269 |
| 1.2 | $Q_2$ | 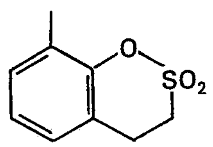 | 189–191 |
| 1.3 | $Q_3$ | 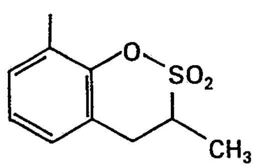 | 186–188 |
| 1.4 | $Q_4$ | 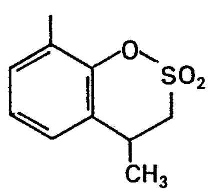 | |
| 1.5 | $Q_6$ | 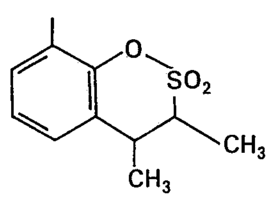 | |
| 1.6 | $Q_6$ | 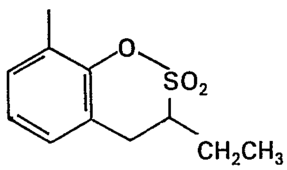 | |

Tabelle 2
$Q{-}SO_2C1$

| Verb. Nr. | Q | Smp. [°C] |
|---|---|---|
| 2.1 | $Q_1$ | 196-204 |
| 2.2 | $Q_2$ | |
| 2.3 | $Q_3$ | |
| 2.4 | $Q_4$ | |
| 2.5 | $Q_5$ | |
| 2.6 | $Q_6$ | |

Tabelle 3
$Q{-}SO_2NCO$

| Verb. Nr. | Q | phys. Daten |
|---|---|---|
| 3.1 | $Q_1$ | |
| 3.2 | $Q_2$ | |
| 3.3 | $Q_3$ | |
| 3.4 | $Q_4$ | |
| 3.5 | $Q_5$ | |
| 3.6 | $Q_6$ | |

Tabelle 4
$Q{-}SO_2{-}NH{-}CO{-}T$

| Verb. Nr. | Q | T | Smp. [°C] |
|---|---|---|---|
| 4.1 | $Q_1$ | $NHCH_3$ | |
| 4.2 | $Q_2$ | $NHCH_3$ | 247–249 |
| 4.3 | $Q_2$ | $NH{-}C_4H_9{-}n$ | |
| 4.4 | $Q_3$ | $NHCH_3$ | |
| 4.5 | $Q_3$ | $NH{-}C_4H_9{-}n$ | |
| 4.6 | $Q_4$ | $NHCH_3$ | |
| 4.7 | $Q_5$ | $NHCH_3$ | |
| 4.8 | $Q_6$ | $NHCH_3$ | |
| 4.9 | $Q_6$ | $NHC_4H_9{-}n$ | |
| 4.10 | $Q_1$ | $OC_6H_5$ | |
| 4.11 | $Q_2$ | $OC_6H_5$ | |
| 4.12 | $Q_3$ | $OC_6H_5$ | |
| 4.13 | $Q_4$ | $OC_6H_5$ | |
| 4.14 | $Q_5$ | $OC_6H_5$ | |
| 4.15 | $Q_6$ | $OC_6H_5$ | |
| 4.16 | $Q_1$ | $OCH_3$ | |
| 4.17 | $Q_2$ | $OCH_3$ | |
| 4.18 | $Q_3$ | $OCH_3$ | |
| 4.19 | $Q_4$ | $OCH_3$ | |
| 4.20 | $Q_6$ | $OCH_3$ | |
| 4.21 | $Q_5$ | $OCH_3$ | |
| 4.22 | $Q_1$ | $OC_2H_5$ | |
| 4.23 | $Q_2$ | $OC_2H_5$ | |
| 4.24 | $Q_3$ | $OC_2H_5$ | |
| 4.25 | $Q_4$ | $OC_2H_5$ | |
| 4.26 | $Q_5$ | $OC_2H_5$ | |
| 4.27 | $Q_6$ | $OC_2H_5$ | |

Tabelle 5

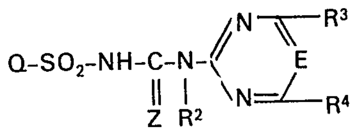

Q hat die in der Tabelle 1 gegebenen Bedeutungen für den anellierten Phenylring.

| Verb. Nr. | Q | $R^2$ | $R^3$ | $R^4$ | Z | E | Smp. [°C] |
|---|---|---|---|---|---|---|---|
| 5.1 | $Q_1$ | H | $CH_3$ | $OCH_3$ | O | N | 210–212 |
| 5.2 | $Q_1$ | H | $OCH_3$ | $OCH_3$ | O | N | |
| 5.3 | $Q_1$ | H | $OCH_3$ | $N(CH_3)_2$ | O | N | |
| 5.4 | $Q_1$ | H | $OCH_3$ | $OCH_2CF_3$ | O | N | |
| 5.5 | $Q_1$ | H | $CH_3$ | $CH_3$ | O | CH | |
| 5.6 | $Q_1$ | H | $OCH_3$ | $CH_3$ | O | CH | 250–253 |
| 5.7 | $Q_1$ | H | $OCH_3$ | $OCH_3$ | O | CH | |
| 5.8 | $Q_1$ | H | $CH_3$ | $OCHF_2$ | O | CH | |
| 5.9 | $Q_2$ | H | $CH_3$ | $OCH_3$ | O | N | 138–187 |
| 5.10 | $Q_2$ | H | $CH_3$ | $CH_3$ | O | N | |
| 5.11 | $Q_2$ | H | $OCH_3$ | $OCH_3$ | O | N | 192–194 |
| 5.12 | $Q_2$ | H | $OCH_3$ | $N(CH_3)_2$ | O | N | |
| 5.13 | $Q_2$ | H | $OCH_3$ | $OCH_2CH_3$ | O | N | |
| 5.14 | $Q_2$ | H | $OCH_3$ | $OCH_2CF_3$ | O | N | |
| 5.15 | $Q_2$ | H | $CH_2CH_3$ | $OCH_3$ | O | N | |
| 5.16 | $Q_2$ | H | $OCH_2CH_3$ | $OCH_2CH_3$ | O | N | |
| 5.17 | $Q_2$ | H | $CH_3$ | $CH_3$ | O | CH | 213–216 |
| 5.18 | $Q_2$ | H | $CH_3$ | $OCH_3$ | O | CH | 218–228 |
| 5.19 | $Q_2$ | H | $CH_3$ | $OCHF_2$ | O | CH | 213–215 |
| 5.20 | $Q_2$ | H | $OCH_3$ | $OCH_3$ | O | CH | 203–205 |
| 5.21 | $Q_2$ | H | $OCH_3$ | $OCHF_2$ | O | CH | |
| 5.22 | $Q_2$ | H | $OCH_3$ | C1 | O | CH | |
| 5.23 | $Q_2$ | H | $OCH_3$ | F | O | CH | |
| 5.24 | $Q_2$ | H | $CH_2F$ | $OCH_3$ | O | CH | |
| 5.25 | $Q_2$ | H | $OCH_3$ | $OCF_2CHF_2$ | O | CH | |
| 5.26 | $Q_2$ | H | $CH_3$ | $OCF_2CHF_2$ | O | CH | |
| 5.27 | $Q_2$ | $CH_3$ | $CH_3$ | $OCH_3$ | O | CH | |
| 5.28 | $Q_2$ | $CH_3$ | $OCH_3$ | $OCH_3$ | O | CH | |
| 5.29 | $Q_2$ | $CH_3$ | $OCH_3$ | $CH_3$ | O | N | |
| 5.30 | $Q_3$ | H | $CH_3$ | $OCH_3$ | O | N | 196–198 |
| 5.31 | $Q_3$ | H | $CH_3$ | $CH_3$ | O | N | |
| 5.32 | $Q_3$ | H | $OCH_3$ | $OCH_3$ | O | N | 129–131 (Zers.) |
| 5.33 | $Q_3$ | H | $OCH_3$ | $N(CH_3)_2$ | O | N | 207–209 |
| 5.34 | $Q_3$ | H | $CH_3$ | $OCH_2CH_3$ | O | N | 187–189 |
| 5.35 | $Q_3$ | H | $OCH_3$ | $OCH_2CF_3$ | O | N | |
| 5.36 | $Q_3$ | H | $CH_2CH_3$ | $OCH_3$ | O | N | 103–106 (Zers.) |
| 5.37 | $Q_3$ | H | $OCH_2CH_3$ | $OCH_2CH_3$ | O | N | 137–140 (Zers.) |
| 5.38 | $Q_3$ | H | $CH_3$ | $CH_3$ | O | CH | 209–210 |
| 5.39 | $Q_3$ | H | $CH_3$ | $OCH_3$ | O | CH | 216–218 |
| 5.40 | $Q_3$ | H | $CH_3$ | $OCHF_2$ | O | CH | 182–184 |
| 5.41 | $Q_3$ | H | $OCH_3$ | $OCH_3$ | O | CH | 202–204 |
| 5.42 | $Q_3$ | H | $OCH_3$ | $OCHF_2$ | O | CH | 105–107 (Zers.) |
| 5.43 | $Q_3$ | H | $OCH_3$ | C1 | O | CH | |
| 5.44 | $Q_3$ | H | $OCH_3$ | F | O | CH | |
| 5.45 | $Q_3$ | H | $CH_2F$ | $OCH_3$ | O | CH | |
| 5.46 | $Q_3$ | H | $OCH_3$ | $OCF_2CHF_2$ | O | CH | |
| 5.47 | $Q_3$ | H | $CH_3$ | $OCF_2CHF_2$ | O | CH | |
| 5.48 | $Q_3$ | $CH_3$ | $OCHF_2$ | $OCHF_2$ | O | CH | 191–193 (Zers.) |
| 5.49 | $Q_3$ | $CH_3$ | $OCH_2$ | $OCH_3$ | O | CH | |
| 5.50 | $Q_3$ | $CH_3$ | $OCH_3$ | $CH_3$ | O | N | |
| 5.51 | $Q_4$ | H | $CH_3$ | $OCH_3$ | O | N | |

Tabelle 5 (Fortsetzung)

| Verb. Nr. | Q | $R^2$ | $R^3$ | $R^4$ | Z | E | Smp. [°C] |
|---|---|---|---|---|---|---|---|
| 5.52 | $Q_4$ | H | $OCH_3$ | $OCH_3$ | O | N | |
| 5.53 | $Q_4$ | H | $OCH_3$ | $N(CH_3)_2$ | O | N | |
| 5.54 | $Q_4$ | H | $OCH_3$ | $OCH_2CF_3$ | O | N | |
| 5.55 | $Q_4$ | H | $CH_3$ | $CH_3$ | O | CH | |
| 5.56 | $Q_4$ | H | $OCH_3$ | $CH_3$ | O | CH | |
| 5.57 | $Q_4$ | H | $OCH_3$ | $OCH_3$ | O | CH | |
| 5.58 | $Q_4$ | H | $CH_3$ | $OCHF_2$ | O | CH | |
| 5.59 | $Q_5$ | H | $CH_3$ | $OCH_3$ | O | N | |
| 5.60 | $Q_5$ | H | $OCH_3$ | $OCH_3$ | O | N | |
| 5.61 | $Q_5$ | H | $OCH_3$ | $N(CH_3)_2$ | O | N | |
| 5.62 | $Q_5$ | H | $OCH_3$ | $OCH_2CF_3$ | O | N | |
| 5.63 | $Q_5$ | H | $CH_3$ | $CH_3$ | O | CH | |
| 5.64 | $Q_5$ | H | $OCH_3$ | $CH_3$ | O | CH | |
| 5.65 | $Q_5$ | H | $OCH_3$ | $OCH_3$ | O | CH | |
| 5.66 | $Q_5$ | H | $CH_3$ | $OCHF_2$ | O | CH | |
| 5.67 | $Q_5$ | H | $CH_3$ | $OCH_3$ | O | N | |
| 5.68 | $Q_5$ | H | $OCH_3$ | $OCH_3$ | O | N | |
| 5.69 | $Q_6$ | H | $OCH_3$ | $N(CH_3)_2$ | O | N | |
| 5.70 | $Q_5$ | H | $OCH_3$ | $OCH_2CF_3$ | O | N | |
| 5.71 | $Q_5$ | H | $CH_3$ | $CH_3$ | O | CH | |
| 5.72 | $Q_6$ | H | $OCH_3$ | $CH_3$ | O | CH | |
| 5.73 | $Q_6$ | H | $OCH_3$ | $OCH_3$ | O | CH | |
| 5.74 | $Q_6$ | H | $CH_3$ | $OCHF_2$ | O | CH | |

Formulierungsbeispiele

Beispiel 4

Formulierungsbeispiele für Wirkstoffe der Formel I

(% = Gewichtsprozent)

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | – | – |
| Na-Diisobutylnaphthalinsulfonat | – | 6% | 6% |
| Octylphenolpolyäthylenglykoläther (7–8 Mol AeO) | – | 2% | 2% |
| Hochdisperse Kieselsäure | 5% | 27% | 27% |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff | 10% | 1% |
| Octylphenolpolyäthylenglykoläther (4–5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

| d) Extruder-Granulat | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff | 40% | 5% |
| Äthylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5% |
| Isopropylamin | 1% |
| Octylphenolpolyäthylenglykoläther (78 Mol AeO) | 3% |
| Wasser | 91% |

Biologische Beispiele

Beispiel 5

Nachweis der Herbizidwirkung vor dem Auflaufen der Pflanzen-Kunststofftöpfe werden mit expandiertem Vermiculit (Dichte: 0,135 g/cm$^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70% gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tage wird dem Giesswasser 0,5% eines handelsüblichen Flüssigdüngers (®Greenzit) zugesetzt. 12 Tage nach der Aussaat wird der Versuch ausgewertet und die Wirkung auf die Versuchspflanzen nach dem folgenden Massstab bewertet:

1 : Pflanze nicht gekeimt oder total abgestorben
2–3: sehr starke Wirkung
4–6: mittlere Wirkung
7–8: schwache Wirkung
9 : keine Wirkung (wie unbehandelte Kontrolle).

Pre-emergente Wirkung: Konzentration der Wirkstoffemulsion: 70,8 ppm

| Testpflanze / Wirkstoff Nr. | Nasturtium | Stellaria | Agrostis | Digitaria |
|---|---|---|---|---|
| 5.9 | 2 | 2 | 1 | 2 |
| 5.18 | 2 | 2 | 2 | 2 |
| 5.19 | 3 | 4 | 2 | 3 |
| 5.20 | 2 | 2 | 2 | 2 |
| 5.30 | 1 | 1 | 1 | 1 |

Beispiel 6

Nachweis der selektiven Herbizidwirkung in Baumwolle vor dem Auflaufen der Pflanzen

Im Gewächshaus werden Samen von Baumwolle und der mit Baumwolle vergesellschafteten Unkräuter in Blumentöpfe von 12–15 cm Durchmesser gesät. Unmittelbar danach wird die Erdoberfläche mit einer wässrigen Dispersion oder Lösung der Wirkstoffe behandelt. Es werden Konzentrationen von 125 und 30 g Wirkstoffmengen pro Hektar angewendet. Die Töpfe werden dann im Gewächshaus bei einer Temperatur von 22–25°C und 50–70% relativer Luftfeuchtigkeit gehalten. Nach 3 Wochen wird der Versuch ausgewertet und die Wirkung nach dem im Beispiel 5 angegebenen Massstab beurteilt.

Beispiel 7

Nachweis der Herbizidwirkung nach dem Auflaufen der Pflanzen (Kontaktwirkung)

Eine Anzahl Unkräuter und Kulturpflanzen, sowohl monokotyle wie dikotyle, werden nach dem Auflaufen, im 4- bis 6-Blatt-Stadium mit einer wässrigen Wirkstoffdispersion in Dosierungen von 0,5 kg/AS/ha gespritzt und dann bei 24° bis 26°C und 45–60% relativer Luftfeuchtigkeit gehalten. 15 Tage nach der Behandlung wird der Versuch ausgewertet und nach dem gleichen Massstab wie im pre-emergenten Versuch bewertet.

Beispiel 8

Nachweis der Wuchshemmung bei tropischen Bodendeckern-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70% relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20% des Neuzuwachses bei unbehandelten Kontrollpflanzen) ohne dass dabei die Versuchspflanzen geschädigt werden.

**Patentansprüche: für die Vertragsstaaten BE CH DE FR GB IT LI NL SE**

1. Anellierte N-Phenylsulfonyl-N'-pyrimidinyl- und triazinyl-harnstoffe der allgemeinen Formel 1 worin

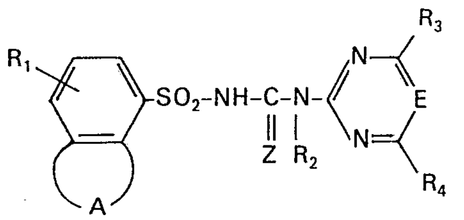

(I),

Z Sauerstoff oder Schwefel,

E Stickstoff oder =CH-,

$R_1$ Wasserstoff, Halogen, Nitro, $C_1$-$C_4$-Alkyl, $C_1$-$C_4$-Halogenalkyl, $C_1$-$C_4$-Alkoxy,$C_1$-$C_4$--Halogenalkoxy, $C_1$-$C_4$-Alkoxycarbonyl, $C_1$-$C_4$-Alkylthio, $C_1$-$C_4$-Alkylsulfinyl, $C_1$-$C_4$-Alkylsulfonyl oder $C_2$-$C_5$-Alkoxyalkoxy,

$R_2$ Wasserstoff, $C_1$-$C_4$-Alkyl oder $C_1$-$C_3$-Alkoxy,

$R_3$ und $R_4$ unabhängig voneinander Wasserstoff, $C_1$-$C_4$-Alkyl, $C_1$-$C_4$-Alkoxy,$C_1$-$C_4$-Halogenalkoxy, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy oder $-NR_5R_6$, wobei $R_5$ und $R_6$ für Wasserstoff oder $C_1$-$C_4$-Alkyl stehen, bedeuten und die Brükke A zusammen mit dem sie tragenden Phenylkern ein 3,4-Dihydro-2,2-dioxo-1,2-benzoxathiin-System bildet, das an der Brücke A gegebenenfalls durch $C_1$-$C_4$-Alkyl substitutiert ist sowie die Salze dieser Verbindungen.

2. Verbindungen gemäss Anspruch 1, dadurch gekennzeichnet, dass Z Sauerstoff bedeutet.

3. Verbindungen gemäss Anspruch 1, dadurch gekennzeichnet, dass $R_1$ Wasserstoff, Halogen oder $C_1$-$C_4$-Alkoxycarbonyl bedeutet.

4. Verbindungen gemäss Anspruch 1, dadurch gekennzeichnet, dass $R_2$ für Wasserstoff steht.

5. Verbindungen gemäss Anspruch 1, dadurch gekennzeichnet, dass $R_3$ und $R_4$ unabhängig voneinander $C_1$-$C_4$-Alkyl, $C_1$-$C_4$-Alkoxy,Di-$C_1$-$C_4$-alkylamino, $C_1$-$C_4$-Halogenalkoxy oder Halogen bedeuten und zusammen höchstens 4 Kohlenstoffatome enthalten.

6. Verbindungen gemäss Anspruch 1, dadurch gekennzeichnet, dass Z Sauerstoff, $R_1$ Wasserstoff, Halogen oder $C_1$-$C_4$-Alkoxycarbonyl, $R_2$ Wasserstoff und $R_3$ und $R_4$ unabhängig voneinander $C_1$-$C_4$-Alkyl, $C_1$-$C_4$-Alkoxy,Di-$C_1$-$C_4$-Alkylamino, $C_1$-$C_4$-Halogenalkoxy oder Halogen, welche zusammen höchstens 4 Kohlenstoffatome enthalten, bedeuten.

7. N-(3,4-Dihydro-2,2-dioxo-3-methyl-1,2-benzoxathiin-8-ylsulfonyl)-N'-(4-methoxy-6-methylpyrimidin-2-yl)-harnstoff gemäss Anspruch 1.

8. N-(3,4-Dihydro-2,2-dioxo-3-methyl-1,2-benzoxathiin-8-ylsulfonyl)-N'-(4,6-dimethoxypyrimidin-2-yl)-harnstoff gemäss Anspruch 1.

9. N-(3,4-Dihydro-2,2-dioxo-3-methyl-1,2-benzoxathiin-8-ylsulfonyl)-N'-(4-methoxy-6-methyl-1,3,5-triazin-2-yl)-harnstoff gemäss Anspruch 1.

10. N-(3,4-Dihydro-2,2-dioxo-3-methyl-1,2-benzoxathiin-8-ylsulfonyl)-N'-(4,6-dimethoxy-1,3,5-triazin-2-yl)harnstoff gemäss Anspruch 1.

11. Anellierte Phenylsulfonamide der allgemeinen Formel II

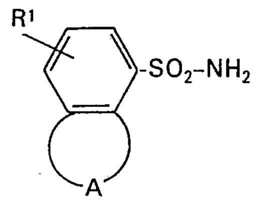

(II),

worin $R_1$ und A die unter Formel I im Anspruch 1 gegebene Bedeutung haben.

12. Anellierte Phenylsulfonylisocyanate und -isothiocyanate der allgemeinen Formel IV

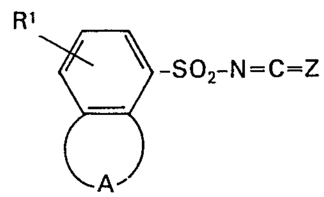

(IV),

worin A, $R_1$, und Z die unter Formel I im Anspruch 1 gegebene Bedeutung haben.

13. Anellierte Phenylsulfonylcarbamate der allgemeinen Formel VI

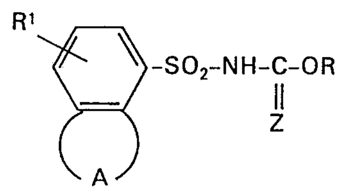

(VI),

worin A, $R_1$ und Z die unter Formel I im Anspruch 1 gegebene Bedeutung haben und R für Phenyl, Alkyl oder substituiertes Phenyl steht.

14. Anellierte Phenylsulfonylchloride der Formel X

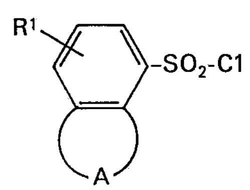

(X),

worin A und $R^1$ die unter Formel I angegebene Bedeutung haben.

15. Verfahren zur Herstellung der Verbindungen der Formel I, gemäss Anspruch 1, dadurch gekennzeichnet, dass man ein anelliertes Phenylsulfonamid der Formel II

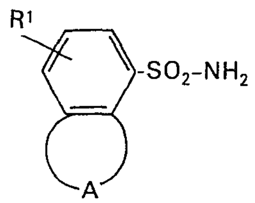

(II),

worin $R_1$ und A die unter Formel I gegebene Bedeutung haben, in Gegenwart einer Base mit einem N-Pyrimidinyl- oder -triazinylcarbamat der Formel III

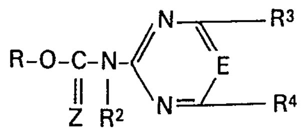

(III)

worin E, $R_2$, $R_3$, $R_4$ und Z die unter Formel I gegebene Bedeutung haben, und T für Phenyl, Alkyl oder substituiertes Phenyl steht, umsetzt und gegebenenfalls in die Salze überführt.

16. Verfahren zur Herstellung der Verbindung der Formel I gemäss Anspruch 1, dadurch gekennzeichnet, dass man ein anelliertes Phenylsulfonylisocyanat oder -isothiocyanat der Formel IV

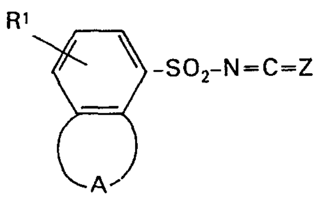

(IV)

worin A, $R_1$ und Z die unter Formel I gegebene Bedeutung haben, gegebenenfalls in Gegenwart einer Base mit einem Aminopyrimidin oder -triazin der Formel V

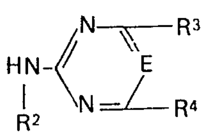

(V)

worin E, $R_2$, $R_3$ und $R_4$ die unter Formel I gegebene Bedeutung haben, umsetzt und gegebenenfalls in die Salze überführt.

17. Verfahren zur Herstellung der Verbindungen der Formel I gemäss Anspruch 1, dadurch gekennzeichnet, dass man ein anelliertes N-Phenylsulfonylcarbamat der Formel VI

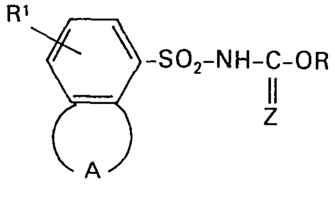

(VI)

worin A, $R_1$ und Z die unter Formel I gegebene Bedeutung haben und R für Phenyl, Alkyl oder substituiertes Phenyl steht, mit einem Aminopyrimidin oder -triazin der oben angegebenen Formel V umsetzt, und gegebenenfalls in die Salze überführt.

18. Verfahren zur Herstellung von Additionssalzen der Formel I gemäss einem der Ansprüche 15 bis 17, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

19. Ein herbizides und den Pflanzenwuchs hemmendes Mittel, dadurch gekennzeichnet, dass es neben Träger- und/oder anderen Zuschlagstoffen als Wirkstoff mindestens einen anellierten N-Phenylsulfonyl-N'-pyrimidinyl- oder -triazinyl-harnstoff der Formel I, Anspruch 1, enthält.

20. Die Verwendung der Wirkstoffe der Formel I, gemäss Anspruch 1, oder sie enthaltender Mittel zur Bekämpfung unerwünschten Pflanzenwachstums.

21. Die Verwendung der Wirkstoffe der Formel I, gemäss Anspruch 1, oder sie enthaltender Mittel zur Hemmung des Pflanzenwachstums.

22. Die Verwendung der Wirkstoffe der Formel I, gemäss Anspruch 1, oder sie enthaltender Mittel zur Beeinflussung des Pflanzenwachstums zum Zweck der Ertragssteigerung.

23. Die Verwendung der Wirkstoffe der Formel I, oder sie enthaltender Mittel, gemäss Anspruch 20, zur selektiven pre- oder postemergenten Bekämpfung von Unkräutern in Nutzpflanzenkulturen.

24. Die Verwendung der N-Arylsulfonyl-N'-pyrimidinyl-harnstoffe der Formel I, oder sie enthaltender Mittel, gemäss Anspruch 21, zur Unterdrückung des Pflanzenwachstums über das 2-Blatt-Stadium hinaus, dadurch gekennzeichnet, dass die Wirkstoffe preemergent angewendet werden.

**Patenansprüche für den Vertragsstaat AT**

1. Ein herbizides und den Pflanzenwuchs hemmendes Mittel, dadurch gekennzeichnet, dass es neben Träger- und/oder anderen Zuschlagstoffen als Wirkstoff mindestens einen anellierten N-Phenylsulfonyl-N'-pyrimidinyl- oder -triazinylharnstoff der Formel I

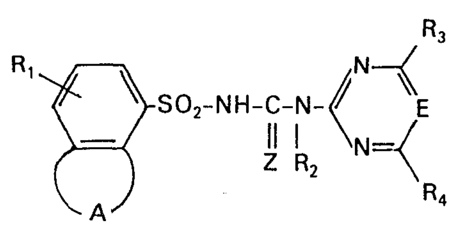

(I)

oder ein Salz davon enthält, worin

Z Sauerstoff oder Schwefel,

E Stickstoff oder =CH-,

$R_1$ Wasserstoff, Halogen, Nitro, $C_1$–$C_4$-Alkyl, $C_1$–$C_4$-Halogenalkyl, $C_1$–$C_4$-Alkoxy, $C_1$–$C_4$-Halogenalkoxy, $C_1$–$C_4$-Alkoxycarbonyl, $C_1$–$C_4$-Alkylthio, $C_1$–$C_4$-Alkylsulfinyl, $C_1$–$C_4$-Alkylsulfonyl oder $C_2$–$C_5$-Alkoxyalkoxy,

$R_2$ Wasserstoff, $C_1$–$C_4$-Alkyl oder $C_1$–$C_3$-Alkoxy,

$R_3$ und $R_4$ unabhängig voneinander Wasserstoff, $C_1$–$C_4$-Alkyl, $C_1$–$C_4$-Alkoxy, $C_1$–$C_4$-Halogenalkoxy, $C_1$–$C_4$-Halogenalkylthio, $C_1$–$C_4$-Alkylthio, Halogen, $C_2$–$C_5$-Alkoxyalkyl, $C_2$–$C_5$-Alkoxyalkoxy oder $-NR_5R_6$, wobei $R_5$ und $R_6$ für Wasserstoff oder $C_1$–$C_4$-Alkyl stehen, bedeuten und die Brükke A zusammen mit dem sie tragenden Phenylkern, ein 3,4-Dihydro-2,2-dioxo-1,2-benzoxathiin-System bildet, das an der Brücke A gegebenenfalls durch $C_1$–$C_4$-Alkyl substituiert ist.

2. Mittel gemäss Anspruch 1, dadurch gekennzeichnet, dass Z Sauerstoff bedeutet.

3. Mittel gemäss Anspruch 1, dadurch gekennzeichnet, dass $R_1$ Wasserstoff, Halogen oder $C_1$–$C_4$-Alkoxycarbonyl bedeutet.

4. Mittel gemäss Anspruch 1, dadurch gekennzeichnet, dass $R_2$ für Wasserstoff steht.

5. Mittel gemäss Anspruch 1, dadurch gekennzeichnet, dass $R_3$ und $R_4$ unabhängig voneinander $C_1$–$C_4$-Alkyl, $C_1$–$C_4$-Alkoxy, Di-$C_1$–$C_4$-alkylamino, $C_1$–$C_4$-Halogenalkoxy oder Halogen bedeuten und zusammen höchstens 4 Kohlenstoffatome enthalten.

6. Mittel gemäss Anspruch 1, dadurch gekennzeichnet, dass Z Sauerstoff, $R_1$ Wasserstoff, Halogen oder $C_1$–$C_4$-Alkoxycarbonyl, $R_2$ Wasserstoff und $R_3$ und $R_4$ unabhängig voneinander $C_1$–$C_4$-Alkyl, $C_1$–$C_4$-Alkoxy, Di-$C_1$–$C_4$-alkylamino, $C_1$–$C_4$-Halogenalkoxy oder Halogen, welche zusammen höchstens 4 Kohlenstoffatome enthalten, bedeuten.

7. Mittel gemäss Anspruch 1, dadurch gekennzeichnet, dass es als Wirkstoff (N-3,4-Dihydro-2,2-dioxo-3-methyl-1,2-benzoxathiin-8-ylsulfonyl)-N'-(4-methoxy-6-methylpyrimidin-2-yl)-harnstoff enthält.

8. Mittel gemäss Anspruch 1, dadurch gekennzeichnet, dass es als Wirkstoff N-(3,4-Dihydro-2,2-dioxo-3-methyl-1,2-benzoxathiin-8-ylsulfonyl)-N'-(4,6-dimethoxypyrimidin-2-yl)-harnstoff enthält.

9. Mittel gemäss Anspruch 1, dadurch gekennzeichnet, dass es als Wirkstoff N-(3,4-Dihydro-2,2-dioxo-3-methyl-1,2-benzoxathiin-8-ylsulfonyl)-N'-(4-methoxy-6-methyl-1,3,5-triazin-2-yl)-harnstoff enthält.

10. Mittel gemäss Anspruch 1, dadurch gekennzeichnet, dass es als Wirkstoff N-(3,4-Dihydro-2,2-dioxo-3-methyl-1,2-benzoxathiin-8-ylsulfonyl)-N'-(4,6-dimethoxy-1,3,5-triazin-2-yl)-harnstoff enthält.

11. Verfahren zur Herstellung der Verbindungen der Formel I, gemäss Anspruch 1, dadurch gekennzeichnet, dass man entweder

a) ein anelliertes Phenylsulfonamid der Formel II

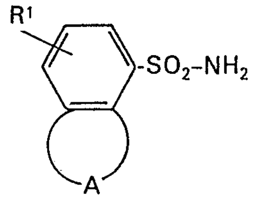

(II)

worin $R_1$ und A die unter Formel I gegebene Bedeutung haben, in Gegenwart einer Base mit einem N-Pyrimidinyl- oder -triazinylcarbamat der Formel III

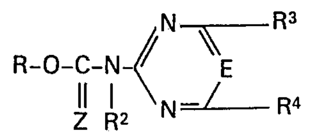

(III)

worin E, $R_2$, $R_3$, $R_4$ und Z die unter Formel I gegebene Bedeutung haben, und T für Phenyl, Alkyl oder substituiertes Phenyl steht, umsetzt oder

b) ein anelliertes Phenylsulfonylisocyanat oder -isothiocyanat der Formel IV

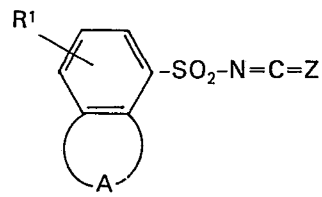

(IV)

worin A, $R_1$ und Z die unter Formel I gegebene Bedeutung haben, gegebenenfalls in Gegenwart einer Base mit einem Aminopyrimidin oder -triazin der Formel V

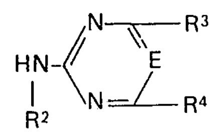

(V)

worin E, $R_2$, $R_3$ und $R_4$ die unter Formel I gegebene Bedeutung haben, umsetzt oder

c) ein anelliertes N-Phenylsulfonylcarbamat der Formel VI

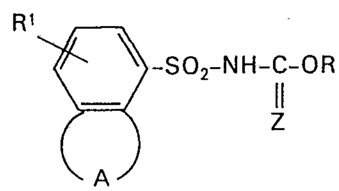

(VI)

worin A, $R_1$ und Z die unter Formel I gegebene Bedeutung haben und R für Phenyl, Alkyl oder substituiertes Phenyl steht, mit einem Aminopyrimidin oder -triazin der oben angegebenen Formel V umsetzt, und gegebenenfalls in die Salze überführt, indem man einen Sulfonylharnstoff der Formel I mit einem Amin, einem Alkalimetall- oder Erdalkalimetallhydroxid oder einer quaternären Ammoniumbase umsetzt.

12. Die Verwendung der Wirkstoffe der Formel I, gemäss Anspruch 1, oder sie enthaltender Mittel zur Bekämpfung unerwünschten Pflanzenwachstums.

13. Die Verwendung der Wirkstoffe der Formel I, gemäss Anspruch 1, oder sie enthaltender Mittel zur Hemmung des Pflanzenwachstums.

14. Die Verwendung der Wirkstoffe der Formel I, gemäss Anspruch 1, oder sie enthaltender Mittel zur Beeinflussung des Pflanzenwachstums zum Zweck der Ertragssteigerung.

15. Die Verwendung der Wirkstoffe der Formel I, oder sie enthaltender Mittel, gemäss Anspruch 12, zur selektiven pre- oder postemergenten Bekämpfung von Unkräutern in Nutzpflanzenkulturen.

16. Die Verwendung der N-Arylsulfonyl-N′-pyrimidinyl-harnstoffe der Formel I, oder sie enthaltender Mittel, gemäss Anspruch 13, zur Unterdrückung des Pflanzenwachstums über das 2-Blatt-Stadium hinaus, dadurch gekennzeichnet, dass die Wirkstoffe preemergent angewendet werden.

**Claims for the Contracting States: DE, GB, FR, CH, LI, IT, NL, BE, SE**

1. A fused N-phenylsulfonyl-N′-pyrimidinyl-urea or N-phenylsulfonyl-N′-triazinylurea of the general formula I

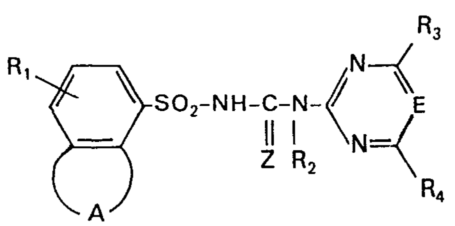

(I)

wherein

Z is oxygen or sulfur,

E is nitrogen or =CH–,

$R_1$ is hydrogen, halogen, nitro, $C_1$–$C_4$ alkyl, $C_1$–$C_4$ haloalkyl, $C_1$–$C_4$ alkoxy, $C_1$–$C_4$ haloalkoxy, $C_1$–$C_4$ alkoxycarbonyl, $C_1$–$C_4$ alkylthio, $C_1$–$C_4$ alkylsulfinyl, $C_1$–$C_4$ alkylsulfonyl or $C_2$–$C_5$ alkoxyalkoxy,

$R_2$ is hydrogen, $C_1$–$C_4$ alkyl or $C_1$–$C_3$ alkoxy,

$R_3$ and $R_4$, each independently of the other, are hydrogen, $C_1$–$C_4$ alkyl, $C_1$–$C_4$ alkoxy, $C_1$–$C_4$ haloalkoxy, $C_1$–$C_4$ haloalkylthio, $C_1$–$C_4$ alkylthio, halogen, $C_2$–$C_5$ alkoxyalkyl, $C_2$–$C_5$ alkoxyalkoxy or $-NR_5R_6$, wherein $R_5$ and $R_6$ are hydrogen or $C_1$–$C_4$ alkyl, and the bridge A, together with the phenyl nucleus which carries it, forms a 3,4-dihydro-2,2-dioxo-1,2-benzoxathiine system which is unsubstituted or subtituted at the bridge A by $C_1$–$C_4$ alkyl, or a salt thereof.

2. A compound according to claim 1, wherein Z is oxygen.

3. A compound according to claim 1, wherein $R_1$ is hydrogen, halogen or $C_1$–$C_4$ alkoxycarbonyl.

4. A compound according to claim 1, wherein $R_2$ is hydrogen.

5. A compound according to claim 1, wherein each of $R_3$ and $R_4$ independently of the other is $C_1$–$C_4$ alkyl, $C_1$–$C_4$ alkoxy, di($C_1$–$C_4$)-alkylamino, $C_1$–$C_4$ haloalkoxy or halogen, and together contain not more than 4 carbon atoms.

6. A compound according to claim 1, wherein Z is oxygen, $R_1$ is hydrogen, halogen or $C_1$–$C_4$ alkoxycarbonyl, $R_2$ is hydrogen, wherein each of $R_3$ and $R_4$ independently of the other is $C_1$–$C_4$ alkyl, $C_1$–$C_4$ alkoxy, di($C_1$–$C_4$) alkylamino, $C_1$–$C_4$ haloalkoxy or halogen, and together contain not more than 4 carbon atoms.

7. N-(3,4-Dihydro-2,2-dioxo-3-methyl-1,2-benzoxathiin-8-ylsulfonyl)-N′-(4-methoxy-6-methylpyrimidin-2-yl)urea according to claim 1.

8. N-(3,4-Dihydro-2,2-dioxo-3-methyl-1,2-benzoxathiin-8-ylsulfonyl)-N′-(4,6-dimethoxypyrimidin-2-yl)urea according to claim 1.

9. N-(3,4-Dihydro-2,2-dioxo-3-methyl-1,2-benzoxathiin-8-ylsulfonyl)-N′-(4-methoxy-6-methyl-1,3,5-triazin-2-yl)urea according to claim 1.

10. N-(3,4-Dihydro-2,2-dioxo-3-methyl-1,2-benzoxathiin-8-ylsulfonyl)-N′-(4,6-dimethoxy-1,3,5-triazin-2-yl)urea according to claim 1.

11. A fused phenylsulfonamide of the general formula II

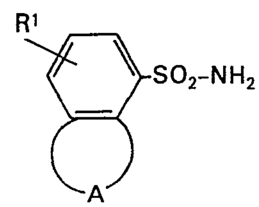

(II)

wherein $R_1$ and A are as defined for formula I in claim 1.

12. A fused phenylsulfonylisocyanate or phenylsulfonylisothiocyanate of the general formula IV

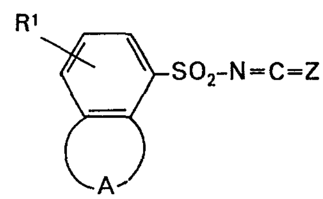

(IV)

wherein A, $R_1$ and Z are as defined for formula I in claim 1.

13. A fused phenylsulfonylcarbamate of the general formula VI

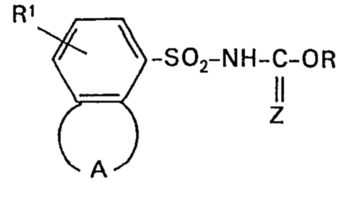

(VI)

wherein A, $R_1$ and Z are as defined for formula I in claim 1, and R is phenyl, alkyl or substituted phenyl.

14. A fused phenylsulfonyl chloride of the formula X wherein A and $R_1$ are as defined for formula I.

15. A process for the preparation of a compound of formula I according to claim 1, which comprises reacting a fused phenylsulfonamide of the formula II

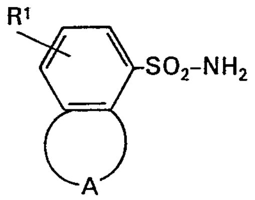
(II)

wherein $R_1$ and A are as defined for formula I, with an N-pyrimidinylcarbamate or N-triazinylcarbamate of the formula III

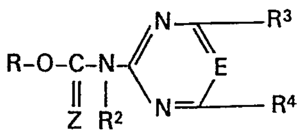
(III)

wherein E, $R_2$, $R_3$, $R_4$ and Z are as defined for formula I and R is phenyl, alkyl or substituted phenyl, in the presence of a base, and, if desired, converting the compound of formula I so obtained into a salt thereof.

16. A process for the preparation of a compound of the formula I according to claim 1, which comprises reacting a fused phenylsulfonylisocyanate or phenylsulfonylisothiocyanate of the formula IV

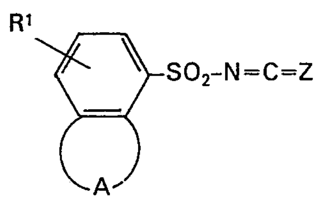
(IV)

wherein A, $R_1$ and Z are as defined for formula I, with an aminopyridine or aminotriazine of the formula V

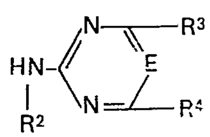
(V)

wherein E, $R_2$, $R_3$ and $R_4$ are as defined for formula I, optionally in the presence of a base, and, if desired, converting the compound of formula I so obtained into a salt thereof.

17. A process for the preparation of a compound of formula I according to claim 1, which comprises reacting a fused N-phenylsulfonylcarbamate of the formula VI

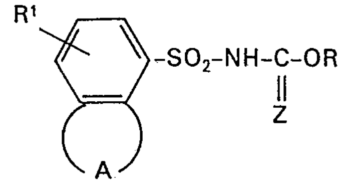
(VI)

wherein A, $R_1$ and Z are as defined for formula I and R is phenyl, alkyl or substituted phenyl, with an aminopyrimidine or aminotriazine of the formula V, and, if desired, converting the compound of formula I so obtained into a salt thereof.

18. A process for the preparation of an addition salt of the formula I according to any one of claims 15 to 17, which comprises reacting a sulfonylurea of the formula I with an amine, an alkali metal hydroxide or alkaline earth metal hydroxide or with a quaternary ammonium base.

19. A herbicidal and plant growth inhibiting composition which contains at least one fused N-phenylsulfonyl-N′-pyrimidinylurea or N-phenylsulfonyl-N′-triazinylurea of the formula I as claimed in claim 1, together with carriers and/or other adjuvants.

20. A method of controlling undesired plant growth, which comprises the use of a compound of the formula I according to claim 1, or of a composition containing such a compound.

21. A method of inhibiting plant growth, which comprises the use of a compound of the formula I according to claim 1, or of a composition containing such a compound.

22. A method of influencing plant growth for increasing yield, which comprises the use of a compound of the formula I according to claim 1, or of a composition containing such a compound.

23. A method according to claim 20 of selectively controlling weeds pre- or postemergence in crops of useful plants, which method comprises the use of a compound of the formula I, or of a composition containing such a compound.

24. A method according to claim 21 of inhibiting plant growth beyond the 2-leaf stage preemergence, which method comprises the use of an N-arylsulfonyl-N′-pyrimidinylurea of the formula I, or of a composition containing such a compound.

**Claims for the Contracting State: AT**

1. A herbicidal and plant growth inhibiting composition which contains as active ingredient at least one fused N-phenylsulfonyl-N′-pyrimidinylurea or N-phenylsulfonyl-N′-triazinylurea of the general formula I

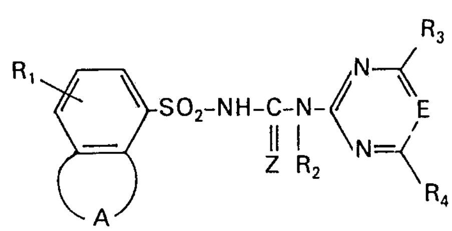
(I)

wherein

Z is oxygen or sulfur,

E is nitrogen or =CH–,

$R_1$ is hydrogen, halogen, nitro, $C_1$–$C_4$ alkyl, $C_1$–$C_4$ haloalkyl, $C_1$–$C_4$ alkoxy, $C_1$–$C_4$ haloalkoxy, $C_1$–$C_4$ alkoxycarbonyl, $C_1$–$C_4$ alkylthio, $C_1$–$C_4$ alkylsulfinyl, $C_1$–$C_4$ alkylsulfonyl or $C_2$–$C_5$ alkoxyalkoxy,

$R_2$ is hydrogen, $C_1$–$C_4$ alkyl or $C_1$–$C_3$ alkoxy,

$R_3$ and $R_4$, each independently of the other, are hydrogen, $C_1$–$C_4$ alkyl, $C_1$–$C_4$ alkoxy, $C_1$–$C_4$ haloalkoxy, $C_1$–$C_4$ haloalkylthio, $C_1$–$C_4$ alkylthio, halogen, $C_2$–$C_5$ alkoxyalkyl, $C_2$–$C_5$ alkoxyalkoxy or $-NR_5R_6$, wherein $R_5$ and $R_6$ are hydrogen or $C_1$–$C_4$ alkyl, and the bridge A, together with the phenyl nucleus which carries it, forms a 3,4-dihydro-2,2-dioxo-1,2-benzoxathiine system which is unsubstituted or substituted at the bridge A by $C_1$–$C_4$ alkyl, or a salt thereof.

2. A composition according to claim 1, wherein Z is oxygen.

3. A composition according to claim 1, wherein $R_1$ is hydrogen, halogen or $C_1$–$C_4$ alkoxycarbonyl.

4. A composition according to claim 1, wherein $R_2$ is hydrogen.

5. A composition according to claim 1, wherein each of $R_3$ and $R_4$ independently of the other is $C_1$–$C_4$ alkyl, $C_1$–$C_4$ alkoxy, di($C_1$–$C_4$) alkylamino, $C_1$–$C_4$ haloalkoxy or halogen, and together contain not more than 4 carbon atoms.

6. A composition according to claim 1, wherein Z is oxygen, $R_1$ is hydrogen, halogen or $C_1$–$C_4$ alkoxycarbonyl, $R_2$ is hydrogen, wherein each of $R_3$ and $R_4$ independently of the other is $C_1$–$C_4$ alkyl, $C_1$–$C_4$ alkoxy, di($C_1$–$C_4$) alkylamino, $C_1$–$C_4$ haloalkoxy or halogen, and together contain not more than 4 carbon atoms.

7. A composition according to claim 1, which contains as active ingredient N-(3,4-dihydro-2,2-dioxo-3-methyl-1,2-benzoxathiin-8-ylsulfonyl)-N′-(4-methoxy-6-methylpyrimidin-2-yl)urea.

8. A composition according to claim 1, which contains as active ingredient N-(3,4-dihydro-2,2-dioxo-3-methyl-1,2-benzoxathiin-8-ylsulfonyl)-N′-(4,6-dimethoxypyrimidin-2-yl)urea.

9. A composition according to claim 1, which contains as active ingredient N-(3,4-dihydro-2,2-dioxo-3-methyl-1,2-benzoxathiin-8-ylsulfonyl)-N′-(4-methoxy-6-methyl-1,3,5-triazin-2-yl) urea.

10. A composition according to claim 1, which contains as active ingredient N-(3,4-dihydro-2,2-dioxo-3-methyl-1,2-benzoxathiin-8-ylsulfonyl)-N′-(4,6-dimethoxy-1,3,5-triazin-2-yl)urea.

11. A process for the preparation of a compound of formula I according to claim 1, which comprises either

a) reacting a fused phenylsulfonamide of the formula II

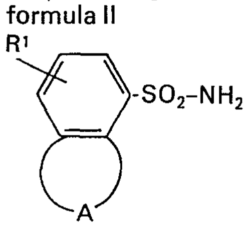

(II)

wherein $R_1$ and A are as defined for formula I, with an N-pyrimidinylcarbamate or N-triazinylcarbamate of the formula III

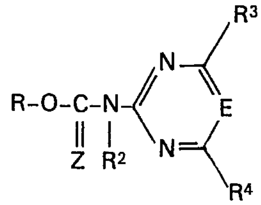

(III).

wherein E, $R_2$, $R_3$, $R_4$ and Z are as defined for formula I and R is phenyl, alkyl or substituted phenyl, in the presence of a base, or

b) reacting a fused phenylsulfonylisocyanate or phenylsulfonylisothiocyanate of the formula IV

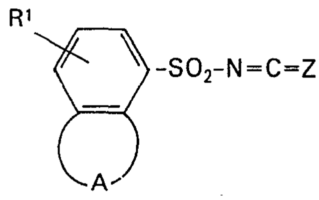

(IV)

wherein A, $R_1$ and Z are as defined for formula I, with an aminopyridine or aminotriazine of the formula V

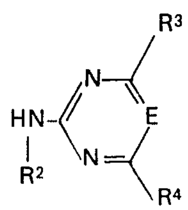

(V)

wherein E, $R_2$, $R_3$ and $R_4$ are as defined for formula I, optionally in the presence of a base, or

c) reacting a fused N-phenylsulfonylcarbamate of the formula VI

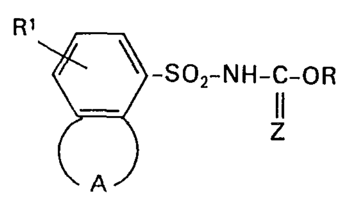

(VI)

wherein A, $R_1$ and Z are as defined for formula I and R is phenyl, alkyl or substituted phenyl, with an aminopyrimidine or aminotriazine of the formula V, and, if desired, converting the sulfonylurea of formula I

so obtained into a salt thereof by reacting said sulfonylurea of formula I with an amine, an alkali metal hydroxide or alkaline earth metal hydroxide or with a quaternary ammonium base.

12. A method of controlling undesired plant growth, which comprises the use of a compound of the formula I according to claim 1, or of a composition containing such a compound.

13. A method of inhibiting plant growth, which comprises the use of a compound of the formula I according to claim 1, or of a composition containing such a compound.

14. A method of influencing plant growth for increasing yield, which comprises the use of a compound of the formula I according to claim 1, or of a composition containing such a compound.

15. A method according to claim 12 of selectively controlling weeds pre- or postemergence in crops of useful plants, which method comprises the use of a compound of the formula I, or of a composition containing such a compound.

16. A method according to claim 13 of inhibiting plant growth beyond the 2-leaf stage preemergence, which method comprises the use of an N-arylsulfonyl-N'-pyrimidinylurea of the formula I, or of a composition containing such a compound.

**Revendications pour les Etats contractants: BE, CH, DE, FR, GB, IT, LI, NL, SE**

1. N-phénylsulfonyl-N'-pyrimidinyl- et -triazinyl-urées condensées de formule générale I

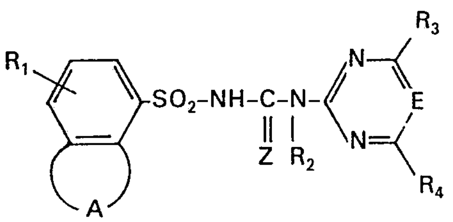
(I)

dans laquelle

Z représente l'oxygène ou le soufre,

E représente l'azote ou =CH–,

$R_1$ représente l'hydrogène, un halogène, un groupe nitro, alkyle en $C_1$–$C_4$, halogénoalkyle en $C_1$–$C_4$, alcoxy en $C_1$–$C_4$, halogénoalcoxy en $C_1$–$C_4$, (alcoxy en $C_1$–$C_4$)-carbonyle, alkylthio en $C_1$–$C_4$, alkylsulfinyle en $C_1$–$C_4$, alkylsulfonyle en $C_1$–$C_4$ ou alcoxyalcoxy en $C_2$–$C_5$,

$R_2$ représente l'hydrogène, un groupe alkyle en $C_1$–$C_4$ ou alcoxy en $C_1$–$C_3$,

$R_3$ et $R_4$ représentent chacun, indépendamment l'un de l'autre, l'hydrogène, un groupe alkyle en $C_1$–$C_4$, alcoxy en $C_1$–$C_4$, halogénoalcoxy en $C_1$–$C_4$, halogénoalkylthio en $C_1$–$C_4$, alkylthio en $C_1$–$C_4$, un halogène, un groupe alcoxyalkyle en $C_2$–$C_5$, alcoxyalcoxy en $C_2$–$C_5$ ou $-NR_5R_6$ dans lequel $R_5$ et $R_6$ représentent l'hydrogène ou des groupes alkyle en $C_1$–$C_4$, et le pont A forme, avec le noyau phényle qui le porte, un système 3,4-dihydro-2,2-dioxo-1,2-benzoxathiine éventuellement substitué sur le pont A par un groupe alkyle en $C_1$–$C_4$, et les sels de ces composés.

2. Composés selon la revendication 1, caractérisés en ce que Z représente l'oxygène.

3. Composés selon la revendication 1, caractérisés en ce que $R_1$ représente l'hydrogène, un halogène ou un groupe (alcoxy en $C_1$–$C_4$)-carbonyle.

4. Composés selon la revendication 1, caractérisés en ce que $R_2$ représente l'hydrogène.

5. Composés selon la revendication 1, caractérisés en ce que $R_3$ et $R_4$ représentent chacun, indépendamment l'un de l'autre, un groupe alkyle en $C_1$–$C_4$, alcoxy en $C_1$–$C_4$, di-(alkyle en $C_1$–$C_4$)-amino, halogénoalcoxy en $C_1$–$C_4$ ou un halogène et contiennent ensemble 4 atomes de carbone au maximum.

6. Composés selon la revendication 1, caractérisés en ce que Z représente l'oxygène, $R_1$ l'hydrogène, un halogène ou un groupe (alcoxy en $C_1$–$C_4$)-carbonyle, $R_2$ représente l'hydrogène, et $R_3$ et $R_4$ représentent chacun, indépendamment l'un de l'autre, un groupe alkyle en $C_1$–$C_4$, alcoxy en $C_1$–$C_4$, di-(alkyle en $C_1$–$C_4$)-amino, halogénoalcoxy en $C_1$–$C_4$ ou un halogène, et contiennent ensemble au maximum 4 atomes de carbone.

7. La N-(3,4-dihydro-2,2-dioxo-3-méthyl-1,2-benzozathiine-8-ylsulfonyl)-N'-(4-méthoxy-6-méthylpyrimidine-2-yl)-urée selon la revendication 1.

8. La N-(3,4-dihydro-2,2-dioxo-3-méthyl-1,2-benzoxathiine-8-ylsulfonyl)-N'-(4,6-diméthoxypyrimidine-2-yl)-urée selon la revendication 1.

9. La N-(3,4-dihydro-2,2-dioxo-3-méthyl-1,2-benzoxathiine-8-ylsulfonyl)-N'-(4-méthoxy-6-méthyl-1,3,5-triazine-2-yl)-urée selon la revendication 1.

10. La N-(3,4-dihydro-2,2-dioxo-3-méthyl-1,2-benzoxathiine-8-ylsulfonyl)-N'-(4,6-diméthoxy-1,3,5-triazine-2-yl)-urée selon la revendication 1.

11. Phénylsulfonamides condensés de formule générale II

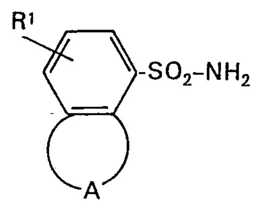
(II)

dans laquelle $R_1$ et A ont les significations indiquées dans la revendication 1 en référence à la formule I.

12. Phénylsulfonylisocyanates et -isothiocyanates condensés de formule générale IV

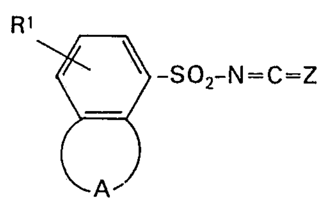
(IV)

dans laquelle A, $R_1$ et Z ont les significations indiquées dans la revendication 1 en référence à la formule I.

13. Phénylsulfonylcarbamates condensés de formule générale VI

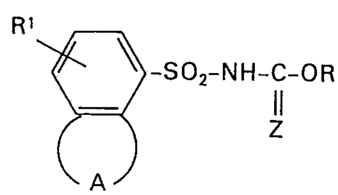
(VI)

dans laquelle A, $R_1$ et Z ont les significations indiquées dans la revendication 1 en référence à la formule I et R représente un groupe phényle, alkyle ou phényle substitué.

14. Chlorures de phénylsulfonyle condensés de formule X

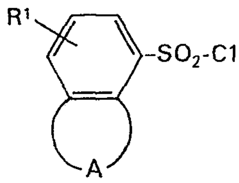

(X),

dans laquelle A et $R^1$ ont les significations indiquées en référence à la formule I.

15. Procédé de préparation des composés de formule I selon la revendication 1, caractérisé en ce que l'on fait réagir un phénylsulfonamide condensé de formule II

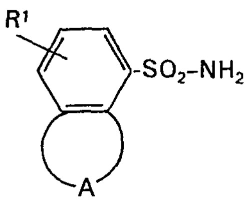

(II)

dans laquelle $R_1$ et A ont les significations indiquées en référence à la formule I, en présence d'une base, avec un N-pyrimidinyl- ou -triazinylcarbamate de formule III

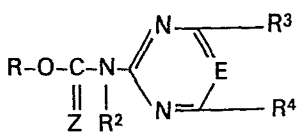

(III)

dans laquelle E, $R_2$, $R_3$, $R_4$ et Z ont les significations indiquées en référence à la formule I et T représente un groupe phényle, alkyle ou phényle substitué, et le cas échéant on convertit en les sels.

16. Procédé de préparation des composés de formule I selon la revendication 1, caractérisé en ce que l'on fait réagir un phénylsulfonylisocyanate ou -isothiocyanate condensé de formule IV

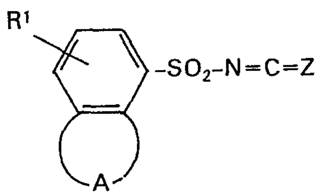

(IV)

dans laquelle A, $R_1$ et Z ont les significations indiquées en référence à la formule I, éventuellement en présence d'une base, avec une aminopyrimidine ou -triazine de formule V

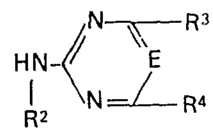

(V)

dans laquelle E, $R_2$, $R_3$ et $R_4$ ont les significations indiquées en référence à la formule I, et le cas échéant on convertit en les sels.

17. Procédé de préparation des composés de formule I selon la revendication 1, caractérisé en ce que l'on fait réagir un N-phénylsulfonylcarbamate condensé de formule VI

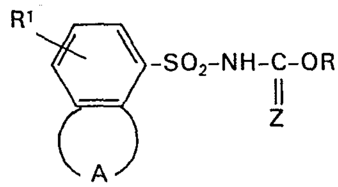

(VI)

dans laquelle A, $R^1$ et Z ont les significations indiquées en référence à la formule I et R représente un groupe phényle, alkyle ou phényle substitué, avec une aminopyrimidine ou -triazine de formule V ci-dessus, et le cas échéant en convertit en les sels.

18. Procédé de préparation des sels d'addition de formule I selon l'une des revendications 15 à 17, caractérisé en ce que l'on fait réagir une sulfonylurée de formule I avec une amine, un hydroxyde de métal alcalin ou de métal alcalino-terreux ou une base d'ammonium quaternaire.

19. Un produit herbicide et inhibiteur de la croissance des végétaux, caractérisé en ce qu'il contient en tant que substance active, avec des véhicules et/ou d'autres additifs, au moins une N-phénylsulfonyl-N'-pyrimidinyl- ou -triazinylurée condensée de formule I, revendication 1.

20. L'utilisation des substances actives de formule I selon la revendication 1 ou de produits en contenant dans la lutte contre les croissances de végétaux indésirables.

21. L'utilisation des substances actives de formule 1 selon la revendication 1 ou de produits en contenant pour l'inhibition de la croissance des végétaux.

22. L'utilisation des substances actives de formule I selon la revendication 1 ou de produits en contenant pour agir sur la croissance des végétaux en vue de parvenir à une augmentation de la récolte.

23. L'utilisation des substances actives de formule I ou de produits en contenant selon la revendication 20, pour la lutte sélective en prélevée ou en post-levée contre les mauvaises herbes dans les cultures de végétaux utiles.

24. L'utilisation des N-arylsulfonyl-N'-pyrimidinyl-urées de formule I ou de produits en contenant selon la revendication 21, pour l'inhibition de la croissance des végétaux au-delà du stade deux feuilles, caractérisée en ce que l'on applique les substances actives en pré-levée.

**Revendications pour l'Etat contractant: AT**

1. Un produit herbicide et inhibiteur de la croissance des végétaux, caractérisé en ce qu'il contient en tant que substance active, avec des véhicules et/ou d'autres additifs, au moins une N-phénylsulfonyl-N'-pyrimidinyl- ou -triazinyl-urée condensée de formule I

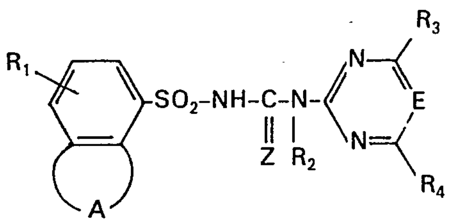

(I)

ou un sel d'un tel composé, dans lesquels

Z représente l'oxygène ou le soufre,

E représente l'azote ou =CH–,

$R_1$ représente l'hydrogène, un halogène, un groupe nitro, alkyle en $C_1$–$C_4$, halogénoalkyle en $C_1$–$C_4$, alcoxy en $C_1$–$C_4$, halogénoalcoxy en $C_1$–$C_4$, (alcoxy en $C_1$–$C_4$)-carbonyle, alkylthio en $C_1$–$C_4$, alkylsulfinyle en $C_1$–$C_4$, alkylsulfonyle en $C_1$–$C_4$ ou alcoxyalcoxy en $C_2$–$C_5$,

$R_2$ représente l'hydrogène, un groupe alkyle en $C_1$–$C_4$ ou alcoxy en $C_1$–$C_3$,

$R_3$ et $R_4$ représentent chacun, indépendamment l'un de l'autre, l'hydrogène, un groupe alkyle en $C_1$–$C_4$, alcoxy en $C_1$–$C_4$, halogénoalcoxy en $C_1$–$C_4$, halogénoalkylthio en $C_1$–$C_4$, alkylthio en $C_1$–$C_4$, un halogène, un groupe alcoxyalkyle en $C_2$–$C_5$, alcoxyalcoxy en $C_2$–$C_5$ ou $-NR_5R_6$ dans lequel $R_5$ et $R_6$ représentent l'hydrogène ou des groupes alkyle en $C_1$–$C_4$, et le pont A forme avec le noyau phényle qui le porte un système 3,4-dihydro-2,2-dioxo-1,2-benzoxathiine qui est éventuellement substitué sur le pont A par un groupe alkyle en $C_1$–$C_4$.

2. Produit selon la revendication 1, caractérisé en ce que Z représente l'oxygène.

3. Produit selon la revendication 1, caractérisé en ce que $R_1$ représente l'hydrogène, un halogène ou un groupe (alcoxy en $C_1$–$C_4$)-carbonyle.

4. Produit selon la revendication 1, caractérisé en ce que $R_2$ représente l'hydrogène.

5. Produit selon la revendication 1, caractérisé en ce que $R_3$ et $R_4$ représentent chacun, indépendamment l'un de l'autre, un groupe alkyle en $C_1$–$C_4$, alcoxy en $C_1$–$C_4$, di-(alkyle en $C_1$–$C_4$)-amino, halogénoalcoxy en $C_1$–$C_4$ ou un halogène et contiennent ensemble 4 atomes de carbone au maximum.

6. Produit selon la revendication 1, caractérisé en ce que Z représente l'oxygène, $R_1$ représente l'hydrogène, un halogène ou un groupe (alcoxy en $C_1$–$C_4$)-carbonyle, $R_2$ représente l'hydrogène et $R_3$ et $R_4$ représentent chacun, indépendamment l'un de l'autre, un groupe alkyle en $C_1$–$C_4$, alcoxy en $C_1$–$C_4$, di-(alkyle en $C_1$–$C_4$)-amino, halogénoalcoxy en $C_1$–$C_4$ ou un halogène et contiennent ensemble au maximum 4 atomes de carbone.

7. Produit selon la revendication 1, caractérisé en ce qu'il contient en tant que substance active la N-(3,4-dihydro-2,2-dioxo-3-méthyl-1,2-benzoxathiine-8-ylsulfonyl)-N'-(4-méthoxy-6-méthylpyrimidine-2-yl)-urée.

8. Produit selon la revendication 1, caractérisé en ce qu'il contient en tant que substance active la N-(3,4-dihydro-2,2-dioxo-3-méthyl-1,2-benzoxathiine-8-ylsulfonyl)-N'-(4,6-diméthoxypyrimidine-2-yl)-urée.

9. Produit selon la revendication 1, caractérisé en ce qu'il contient en tant que substance active la N-(3,4-dihydro-2,2-dioxo-3-méthyl-1,2-benzoxathiine-8-ylsulfonyl)-N'-(4-méthoxy-6-méthyl-1,3,5-triazine-2-yl)-urée.

10. Produit selon la revendication 1, caractérisé en ce qu'il contient en tant que substance active la N-(3,4-dihydro-2,2-dioxo-3-méthyl-1,2-benz-oxathiine-8-ylsulfonyl)-N'-(4,6-diméthoxy-1,3,5-triazine-2-yl)-urée.

11. Procédé de préparation des composés de formule I selon la revendication 1, caractérisé en ce que

a) on fait réagir un phénylsulfonamide condensé de formule II

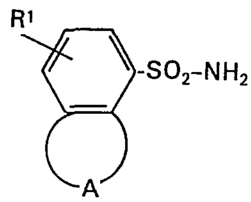

(II)

dans laquelle $R_1$ et A ont les significations indiquées en référence à la formule I, en présence d'une base, avec un N-pyrimidinyl- ou -triazinyl-carbamate de formule III

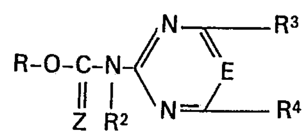

(III)

dans laquelle E, $R_2$, $R_3$, $R_4$ et Z ont les significations indiquées en référence à la formule I, et T représente un groupe phényle, alkyle ou phényle substitué, ou bien

b) on fait réagir un phénylsulfonylisocyanate ou -isothiocyanate condensé de formule IV

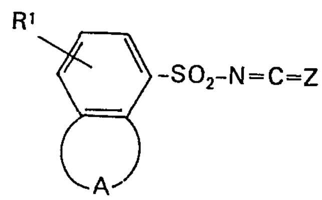

(IV)

dans laquelle A, $R_1$ et Z ont les significations indiquées en référence à la formule I, éventuellement en présence d'une base, avec une aminopyrimidine ou -triazine de formule V

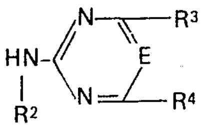

(V)

dans laquelle E, $R_2$, $R_3$ et $R_4$ ont les significations indiquées en référence à la formule I, ou bien

c) on fait réagir un N-phénylsulfonylcarbamate condensé de formule VI

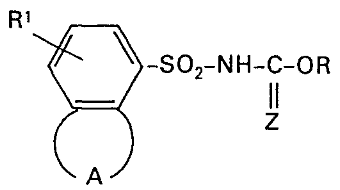

(VI)

dans laquelle A, $R_1$ et Z ont les significations indiquées en référence à la formule I et R représente un groupe phényle, alkyle ou phényle substitué, avec une aminopyrimidine ou -triazine de formule V ci-dessus, et le cas échéant on convertit en les sels par réaction d'une sulfonylurée de formule I avec une amine, un hydroxyde de métal alcalin ou alcalino-terreux ou une base d'ammonium quaternaire.

12. L'utilisation des substances actives de formule I selon la revendication 1 ou de produits en contenant dans la lutte contre les croissances de végétaux indésirables.

13. L'utilisation des substances actives de formule I selon la revendication 1 ou de produits en contenant pour l'inhibition de la croissance des végétaux.

14. L'utilisation des substances actives de formule I selon la revendication 1 ou de produits en contenant pour agir sur la croissance des végétaux en vue d'augmenter la récolte.

15. L'utilisation des substances actives de formule I ou de produits en contenant selon la revendication 12, pour la lutte sélective en prélevée ou en post-levée contre les mauvaises herbes dans les cultures de végétaux utiles.

16. L'utilisation des N-arylsulfonyl-N'-pyrimidinyl-urées de formule I ou de produits en contenant selon la revendication 13, pour l'inhibition de la croissance des végétaux au-delà du stade deux feuilles, caractérisée en ce que l'on applique les substances actives en pré-levée.